(19) Europäisches Patentamt
European Patent Office
Office européen des brevets

(11) Publication number: 0 163 806
B1

(12) EUROPEAN PATENT SPECIFICATION

(45) Date of publication of patent specification: 29.08.90

(21) Application number: 85100768.2

(22) Date of filing: 25.01.85

(51) Int. Cl.$^5$: C 07 C 69/003, C 07 C 69/33, C 07 C 69/58, C 07 C 67/00, A 61 K 7/48

# (54) Mixtures of oligomers of partially esterified pentaerythritol, their preparation and use.

(30) Priority: 07.05.84 US 607694

(43) Date of publication of application: 11.12.85 Bulletin 85/50

(45) Publication of the grant of the patent: 29.08.90 Bulletin 90/35

(84) Designated Contracting States: BE CH DE FR GB IT LI NL SE

(56) References cited:
EP-A-0 100 652
DE-B-1 200 795
US-A-3 670 013

Patent Abstracts of Japan, unexamined applications, section C, vol. 3, no. 128(-62), October 24, 1979 The Patent Office Japanese Government page 23 C 62

(73) Proprietor: MIRANOL INC.
68 Culver Road
Dayton New Jersey (US)

(72) Inventor: Nadolsky, Richard J.
Paint Island Spring Road
Clarksburg New Jersey (US)
Inventor: Fridella, Dominick
40 Whittier Road
Clark New Jersey (US)
Inventor: Laryea, Joseph M.
14 Glen Avenue
Old Bridge New Jersey (US)

(74) Representative: Baillie, Iain Cameron et al
c/o Ladas & Parry Isartorplatz 5
D-8000 München 2 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 163 806 B1

**Description**

1. General Subject of the Invention

The present invention relates to oligomers of partially esterified pentaerythritol, their production and use in cosmetics and toiletries and as lubricants and corrosion inhibitors.

2. Prior Art

Esters of polypentaerythritols have been known for many years. For examle, U.S. Patent 2,686,766 (Silverstein et al issued August 17, 1954) describes the use of tripentaerythritol which has been partially esterified with a fatty acid as defoaming agents in synthetic rubber latex base water emulsion paints. A typical product is obtained by reaction of tripentaerythritol with methyl laurate in the presence of a base.

U.S. Patent 2,958,706 (Hurwitz et al issued November 1, 1960) describes the substantially complete esterification of mixture of polypentaerythritol with fatty acids of 4—8 carbon atoms. The products have a hydroxyl value less than 5 and are useful in plasticising polyvinyl chloride.

U.S. Patent 2,975,152 (Hurwitz et al issued November 1, 1961) describes the esterification of pentaerythritols and polypentaerythritols to produce plasticisers for polyvinylchloride.

U.S. Patent 4,421,565 (DiBella issued December 20, 1983) describes a thixotropic agent obtained by reacting a mixture of polypentaerythritols with a fatty acid of 20 to 22 carbon atoms. Reference is, however, made to the possibility of using as the fatty acid, "behenic acid", a commercial mixture of fatty acids consisting predominantly of $C_{20}$ and $C_{22}$ acids but containing less than 10% $C_{18}$ acids.

U.S. Patent 3,670,013 (Liepfried issued June 13, 1972) discloses a process for partially esterifying neopentyl polyols (such as pentaerythritol) and then condensing the product to form oligomers. The products are stated to be useful as intermediates to fully esterified products that are useful as plasticizers for synthetic resins or as base stocks for synthetic lubricants. It is taught that the reaction should take place under a blanket of low-boiling azeotrope forming acids which inevitably participate in the esterification reaction thereby leading to the presence of short chain residues (e.g. of 5—7 carbon atoms) in the product. The sole example indicates that a typical degree of oligomerization as to produce oligomers having an average of less than two neopentyl units.

In European Patent Publication A151992, there are described partial esters of tripentaerythritol of a fatty acid. These products are soft solids and have moisturizing and emollient properties and are useful in toiletries and cosmetics. A similar disclosure is found in Patent Abstracts of Japan Kokai No. 54—106415.

In the acid catalyzed esterification of pentaerythritol using from 0.5 to 2.5 moles of fatty acid per mole of pentaerythritol, we have surprisingly found that after removal of the required amount of water to form partial esters of pentaerythritol further elimination of water occurs readily under the same conditions employed for esterification. This further reaction yields novel condensation products which are apparently oligomers of the pentaerythritol partial esters.

These products differ from those obtained by esterification of polypentaerythritol (described in European Patent Publication A151992 and Japanese Patent Abstract Kokai 54—106415 in that, for a given fatty acid at a given ratio of fatty acid to contained pentaerythritol, the oligomeric products described herein are more liquid than those derived from a polypentaerythritol. As shown by examples 8, 9 and 10 hereof the products of the present invention have superior properties to the partially esterified polypentaerythritols. Without wishing to be bound by any theory, we believe a possible explanation for this difference may lie in the fact that the products produced in the present case are mixtures of oligomers rather than a single oligomer, and that the distribution of ester groups is more random in the present case than in products made from a polypentaerythritol.

Summary of the Invention

From a first aspect, the present invention provides a mixture of oligomers of pentaerythritol which has been esterified with from 0.5 to 2.5 moles of a saturated or unsaturated fatty acid of 6 to 18 carbon atoms, for example 12 to 17 carbon atoms preferably 12 to 16 carbon atoms per mole of pentaerythritol wherein said mixture of oligomers contains an average of 2 to 7 partially esterified pentaerythritol units per oligomer chain.

From a second aspect, the present invention provides a process for the production of such oligomers which comprises esterifying pentaerythritol with from 0.5 to 2.5 moles per mole of pentaerythritol of a saturated or unsaturated fatty acid of from 6 to 18 carbon atoms and condensing said partially esterified pentaerythritol so as to remove from 0.5 to 0.85 moles of water per mole of partially esterified pentaerythritol.

It will be appreciated that the number of moles of water removed determines the average chain length of the oligomer produced. Thus, removal of 0.5 moles of water per mole of partially esterified pentaerythritol results in a product wherein the "average" product contains two pentaerythritol residues whereas removal of 0.85 moles of water per mole of partially esterified pentaerythritol results in a product where the "average" product contains seven pentaerythritol residues.

The condensation reaction produces oligomers of a typical formula

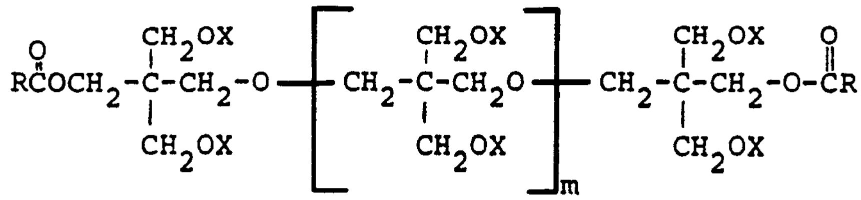

wherein R is a normal saturated or unsaturated alkyl group of 5 to 17 carbon atoms, X is hydrogen or RC(O) — with the proviso that at least one of the X groups is hydrogen and m is an average which is 1 to 5. In any product according to the invention there is a mixture of oligomers of different chain lengths, the distribution of chain length of oligomers about the average chain length typically following a normal distribution.

Particularly preferred compositions according to the present invention are those where the average value of m is 1. That is to say products obtained by the elimination of 0.66 moles of water per mole of partially esterified pentaerythritol. The degree of esterification of the pentaerythritol oligomer is normally in the range 12.5 to 62.5%, preferably in the range 25 to 50%.

Products according to the present invention are prepared by first partially esterifying pentaerythritol with a normal saturated or unsaturated fatty acid of 6 to 18 carbon atoms using from 0.5 to 2.5 moles of fatty acid per mole of pentaerythritol. Typically the fatty acids used are lauric, myristic, palmitic, stearic and oleic acids and mixtures of such acids. The esterification is effected in a suitable solvent that will azeotropically remove the water of reaction and in the presence of a suitable acidic catalyst. Reactions normally are run at temperatures of 150—200°C. Suitable solvents are benzene, toluene, xylene or other solvent that will form an azeotrope with water and allow for achieving a reaction temperature in the range given above. Suitable catalysts are p-toluenesulfonic acid, stannous octoate, or other sufficiently thermally stable Lewis or Bronsted acid. We have, however, found that the partial esterification can in some cases also be effected without the need for a carrier-solvent for removal of water. If a high equivalent weight acid such as p-toluene sulfonic acid is used as catalyst, an excess of this above the amount recorded for catalyst with solvent present will enable the use of a solvent to be avoided. As an alternative, one may use a catalytic amount of a lower equivalent weight acid such as sulfuric acid. Other convenient acids such as methane sulfonic or a mixture of methane sulfonic and hypophosphorous acids may, however, be used to effect the catalysis. The partially esterified pentaerythritol is then condensed to form the oligomers by continued treatment under the same conditions as those used for the partial esterification.

The oligomeric partial esters of pentaerythritol where the average value of m is 1 and the fatty acid is of 12 to 18 preferably 12 to 16 carbon atoms are particularly useful as moisturizers and emollients in cream and lotion formulations. In addition, the oligomeric products, being liquid are easy to work with. The oligomeric products are also useful in lipstick bases and bar soaps. We have found that such oligomers have surprisingly beneficial moisturization and emollient properties. The oligomer mixtures of the present invention especially those of esters of fatty acids of 6—12 carbon atoms also possess lubricating and corrosion-inhibiting properties. Such compositions may be water- or oil-based and typically contain 0.5 to 20%, more commonly 1—10% by weight of the oligomer mixture of the present invention. The corrosion inhibiting compositions are useful in providing protection against acids, alkalis, salts and oxidants.

Cream and lotion formulations normally comprise emulsifiers. Suitable emulsifiers include amine salts of fatty acids of 12 to 20 carbon atoms, for example, alkanolamine salts (e.g. triethanolamine salts) of lauric, myristic, stearic and palmitic acids, mono esters of glycerol with fatty acids of 12 to 20 carbon atoms, such as glycerol monostearate, poly alkoxylated fatty alcohols of 12 to 20 carbon atoms, such as ethoxylates, propoxylates and oleyl alcohol and poly alkoxylate esters such as poly ethoxylates and polypropoxylates) of fatty acids 12 to 20 carbon atoms having from 1 to 20 alkylene oxide units. Such compositions may also contain humectants such as glycerol, sorbitol and $C_2$—$C_4$ alkylene glycols, such as propylene glycol.

Both creams and lotions incorporating the oligomer mixtures of the invention contain substantial quantities of water. The exact physical composition depending upon the ratio of emulsifier to water. We have found that compositions of the present invention required the presence of a lower amount of emulsifier than do prior art moisturizing compositions to ensure a stable cream form of the composition. Typically, skin lotions according to the present invention contain from 65 to 85 percent by weight water whereas creams contain less than 50 percent by weight water.

Such compositions may also contain further components such as emollients. For example, acetylated lanolin alcohols, isopropylmyristate or isopropyl palmitate or mineral oils may be used for this purpose, particularly in moisturizing lotions. However, in view of the emollient properties of the oligomers of the present invention the amount of such emollients present in the compositions of the present invention will be substantially reduced as compared to prior art compounds. For example, the amount of such emollients present in moisturizing creams according to the present invention may be reduced to 5 percent by weight or less.

Other components may also be employed as convenient, for example, stearyl or other fatty alcohols as secondary emulsifiers, materials such as petrolatum and thickeners such as colloidal magnesium aluminum silicate. In particular, it should be noted that the application of compositions according to the present invention is not confined to preparations which are purely moisturizing in function (such as moisturizing creams and lotions) but also find use in other cosmetic preparations where the moisturizing properties of the composition are important such as foundation creams and eyeshadows. Compositions according to the invention which are intended for use in these roles will have the conventional additives used in such compositions, such as cosmetically acceptable pigments.

For example, lipstick, bar soap and makeup foundation compositions may be formulated in accordance with the present invention. Typically, a lipstick composition comprises waxes such as beeswax, carnauba wax and candellila wax together with an emollient such as mineral oil and liquid lanolin base. It also contains pigment normally milled in a liquid base and an opacifier such as titanium dioxide. Foundation makeups typically contain a self emulsifying wax, an emollient such as isopropyl stearate, propylene glycol dipelargonate and opacifiers and pigments such as kaolin and titanium dioxide.

Compositions using the oligomer mixture of the invention typically contain from 2 to 10 percent preferably 2.5 to 5 percent by weight of the oligomers of partially esterified pentaerythritol.

The invention is illustrated by the following Examples in which all percentages are by weight.

### Example 1

To a flask equipped with thermometer, mechanical stirrer, reflux condenser and Dean-Stark trap charge a blend (71/29) of lauric/myristic acid (277.0 g, 1.33 moles), xylene (60 g) and p-toluene-sulfonic acid (1.7 g). Heat, with stirring, to 60°C and bubble a slow stream of nitrogen through the mixture during the entire course of the reaction. At 60°C, add pentaerythritol (136.0 g, 1.0 mole) and heat to 190—195°C over a period of about 1 hour. Care must be taken not to increase temperature at too fast a rate since the pentaerythritol begins to sublime at about 180° by proper control of the rate of temperature increase this sublimation can essentially be avoided and the theoretical amount of water (24 ml, 1.33 moles) collected below 190°C.

When the desired temperature is achieved, continue cooking to remove water resulting in ether formation. When an additional 12 ml of water has been removed, the reaction mixture is cooled below 100°C and the catalyst is neutralized by adding 2.0 g of 25% methanolic sodium methoxide. Methanol and xylene are then stripped off under vacuum at a maximum temperature of 200°C. The product is cooled to 80°C and poured from the flask. After cooling to room temperature, the product is hazy, viscous, yellow liquid.

### Example 2

A product is made by the same procedure as example 1, except the mole ratio of lauric/myristic acid to pentaerythritol is 1.66 to 1.0. This product is also a hazy, viscous liquid.

### Example 3

A product is made by the procedure of example 1 using a 1.0:1.0 mole ratio of lauric/myristic acid to pentaerythritol. The product is a hazy, viscous liquid.

### Example 4

A product is made by the procedure of example 1, except 85% stearic acid is used instead of lauric/myristic acid blend. This product is a hard, cream-colored solid.

### Example 5

A product is made by the procedure of example 1 substituting lauric acid for the lauric/myristic acid blend. This product is a hazy, viscous liquid.

### Example 6

A product is made by the procedure of example 1 using oleic acid instead of the lauric/myristic acid blend. The product is a dark liquid.

### Example 7

A product is made by the procedure of example 1, except that xylene is excluded and sulfuric acid (1.7 g) is used in place of p-toluenesulfonic acid. The product is a hazy, viscous liquid whose performance in the various formulations listed is indistinguishable from that of product made according to example 1.

Example 8
Cleansing Cream

| | A | B |
|---|---|---|
| I. | | |
| Product from Example 1 (batch 1) | 4.0 | — |
| Tripentaerythritol tetra-Laurate/Myristate | — | 4.0 |
| Mineral Oil | 10.0 | 10.0 |
| Cerasynt SD[1] (Van Dyk & Co.) | 5.0 | 5.0 |
| Stearyl Alcohol | 0.5 | 0.5 |
| Cetyl Alcohol | 0.5 | 0.5 |
| II. | | |
| Water | 71.2 | 71.2 |
| Veegum HV[2] (R. T. Vanderbilt) | 0.5 | 0.5 |
| Xanthan Gum (Kelco) | 0.8 | 0.8 |
| III. | | |
| MIRANOL C2M-SF CONC.[3] (Miranol Inc.) | 7.5 | 7.5 |

1. Glyceryl monostearate
2. Colloidal magnesium aluminum silicate thickener
3. Salt-free amphoteric dicarboxylate derived from fatty acid imidazoline.

Procedure:

Mixture I was heated to 75°C. Mixture II was heated with stirring to 80°C until uniform. With agitation, II was added to I and III then added. Agitation was continued until uniform and the composition was then allowed to cool.

Evaluation:

A panel of four was asked to judge these products on the basis of texture, absorbability and feel on the skin. A was chosen over B (4:0).

Example 9
Moisturizing Lotion

| | A | B | C | D | E |
|---|---|---|---|---|---|
| I. | | | | | |
| Product from Example 1 (batch 1) | 5.0 | — | — | — | — |
| Product from Example 1 (batch 2) | — | — | — | 5.0 | — |
| Tripentaerythritol tetra-Laurate/myristate | — | 5.0 | — | — | — |
| Glycerin | — | — | 5.0 | — | — |
| Super Sterol Ester (Croda)[3] | — | — | — | — | 5.0 |
| Arlacel 165[1] (ICI Americas) | 6.0 | 6.0 | 6.0 | 6.0 | 6.0 |
| Mineral Oil | 3.0 | 3.0 | 3.0 | 3.0 | 3.0 |
| Stearic Acid | 2.0 | 2.0 | 2.0 | 2.0 | 2.0 |
| Acetol[2] (Emery Industries) | 1.0 | 1.0 | 1.0 | 1.0 | 1.0 |
| II. | | | | | |
| Water | 78.5 | 78.5 | 78.5 | 78.5 | 78.5 |
| Veegum HV | 0.5 | 0.5 | 0.5 | 0.5 | 0.5 |
| Propylene Glycol | 3.5 | 3.5 | 3.5 | 3.5 | 3.5 |
| Triethanolamine | 0.5 | 0.5 | 0.5 | 0.5 | 0.5 |

1. Glycerol monostearate and polyoxyethylene stearate (Arlacel is a Trade Mark)
2. Acetylated lanolin alcohols
3. $C_{10}$—$C_{30}$ esters of cholesterol and lanolin alcohol

Procedure:

Mixtures I and II were heated separately to 75°C. Mixture II was added to mixture I with agitation. Mixing was continued until uniform, the formulations were then allowed to cool.

Evaluation:

In terms of the performance criteria cited above, a panel of five gave the following ratings:

$$A > D > B \gg C, E$$

Example 10
Night Cream

| | A | B | C |
|---|---|---|---|
| I. | | | |
| Product from Example 1 (batch 1) | 4.0 | — | — |
| Product from Example 1 (batch 2) | — | — | 4.0 |
| Tripentaerythritol tetra-Laurate/ Myristate | — | 4.0 | — |
| Mineral Oil | 25.0 | 25.0 | 25.0 |
| Arlacel 165 | 6.0 | 6.0 | 6.0 |
| Isopropyl Myristate | 5.0 | 5.0 | 5.0 |
| Cetyl Alcohol | 0.5 | 0.5 | 0.5 |
| II. | | | |
| Water | 48.7 | 48.7 | 48.7 |
| Carbopol 934[1] — 3% soln. (B. F. Goodrich) | 5.0 | 5.0 | 5.0 |
| Propylene Glycol | 5.0 | 5.0 | 5.0 |
| III. | | | |
| Triethanolamine | 0.8 | 0.8 | 0.8 |

1. Polyacrylic Acid

Procedure:

Mixture I and II were heated separately to 75°C. Mixture II was then added with agitation to mixture I and III added thereto. Agitation was continued until uniform then the product was allowed to cool.

Evaluation:

On the basis of the previously-cited performance characteristics, a panel of five gave the following ratings:

A, C > B

Example 11
Moisturizing Cream

| | A | B | C |
|---|---|---|---|
| I. | | | |
| Product from Example 1 (batch 1) | 4.0 | — | — |
| Product from Example 1 (batch 2) | — | 4.0 | — |
| Glycerin | — | — | 4.0 |
| Mineral Oil | 3.5 | 3.5 | 3.5 |
| Isopropyl Myristate | 2.0 | 2.0 | 2.0 |
| Propylene Glycol Dipelargonate | 1.0 | 1.0 | 1.0 |
| Arlacel 165 | 5.0 | 5.0 | 5.0 |
| Beeswax | 2.0 | 2.0 | 2.0 |
| Stearic Acid | 1.0 | 1.0 | 1.0 |
| Stearyl Alcohol | 0.5 | 0.5 | 0.5 |
| Dow Corning Fluid 200[1] | 0.5 | 0.5 | 0.5 |
| II. | | | |
| Water | 68.7 | 68.7 | 68.7 |
| Propylene Glycol | 3.5 | 3.5 | 3.5 |
| Carbopol 934 — 3% soln. | 7.5 | 7.5 | 7.5 |
| III. | | | |
| Triethanolamine | 0.8 | 0.8 | 0.8 |

1. A mixture of fully methylated linear siloxane polymers end-blocked with trimethylsiloxy units

Procedure:

Mixtures I and II were heated separately to 75°C. With agitation, mixture II was added to mixture I and component III then added thereto. Agitation was continued until uniform and the product allowed to cool.

Evaluation:

On the basis of previously cited performance criteria, a panel of five gave the following ratings:

A > B (slightly) ≫ C

Example 12
Moisturizing Lotion

| | A | B | C |
|---|---|---|---|
| I. | | | |
| Product from Example 3 | 5.0 | — | — |
| Product from Example 2 | — | 5.0 | — |
| Product from Example 1 (batch 1) | — | — | 5.0 |
| Arlacel 165 | 6.0 | 6.0 | 6.0 |
| Mineral Oil | 3.0 | 3.0 | 3.0 |
| Stearic Acid | 2.0 | 2.0 | 2.0 |
| Acetol | 1.0 | 1.0 | 1.0 |
| II. | | | |
| Water | 78.5 | 78.5 | 78.5 |
| Veegum HV | 0.5 | 0.5 | 0.5 |
| Propylene Glycol | 3.5 | 3.5 | 3.5 |
| Triethanolamine | 0.5 | 0.5 | 0.5 |

Procedure:

Mixtures I and II were heated separately to 75°C. With agitation, mixture II was added to mixture I. Mixing was continued until uniform and the composition then allowed to cool.

Evaluation:

Using previously cited performance criteria, a panel of four gave the following ratings:

C > B (slightly) > A

**Claims**

1. A mixture of oligomers of the formula:

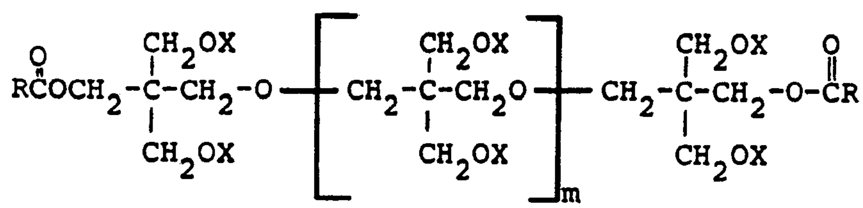

wherein R is a normal saturated or unsaturated alkyl group of 11 to 17 carbon atoms, X is hydrogen or RC(O), with the proviso that at least one of the X groups is hydrogen and the average value of m is 1 to 5.

2. A mixture of oligomers according to claim 1, wherein the average value of m is 1.

3. A mixture of oligomers according to claim 2, wherein an average of from 3 to 5 X groups are hydrogen.

4. A mixture of oligomers according to either of the preceding claims, wherein R is a normal saturated or unsaturated alkyl group of 11 to 15 carbon atoms.

5. A mixture of oligomers according to claims 1—4, wherein RCO is a lauroyl, myristoyl, palmitoyl, stearoyl or oleoyl group.

6. A process for preparing a mixture of oligomers according to claim 1, which comprises esterifying pentaerythritol with from 0.5 to 2.5 moles of a normal saturated or unsaturated fatty acid of from 12 to 18 carbon atoms and subjecting the partial ester product thereof to acid-catalyzed condensation to extract from 0.66 to 0.85 moles of water per mole of pentaerythritol.

7. A process according to claim 6, wherein the condensation is effected to extract about 0.66 moles of water per mole of pentaerythritol.

8. A process according to either of claims 6 and 7, wherein the partial esterification is effected with from 1 to 2 moles per mole of pentaerythritol of an acid selected from lauric, myristic, palmitic, stearic and oleic acids and mixtures thereof.

9. A composition suitable for topical application to the skin which comprises an effective moisturizing or emollient amount of an oligomer mixture of partially esterified pentaerythritol as defined in any one of claims 1 to 5.

10. A composition according to claim 9, which further contains a cosmetically-acceptable emulsifier.

11. A composition according to claim 10, wherein said emulsifier is selected from the group consisting of amine salts of fatty acids, monoesters of glycerol with fatty acid, fatty alcohols of 12 to 20 carbon atoms which are optionally polyalkoxylated and polyalkoxylated esters of fatty acids of 12 to 20 carbon atoms.

12. A composition according to claim 11, wherein said oligomer mixture of partially esterified pentaerythritol is an ester where from 0.5 to 2.5 of the hydroxy groups of the pentaerythritol have been esterified with a fatty acid selected from lauric, myristic, stearic, palmitic and oleic acids.

13. A composition according to claim 12, which further comprises an emollient.

14. A composition according to claim 10, which further comprises an emollient selected from the group comprising acetylated lanolin alcohols, isopropylmyristate and isopropylpalmitate and mineral oil.

**Patentansprüche**

1. Gemisch von Oligomeren der Formel

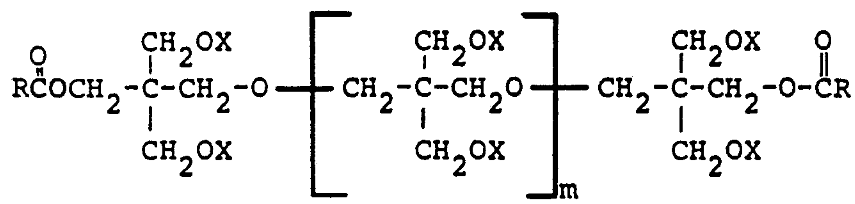

in der R eine normale gesättigte oder ungesättigte Alkylgruppe mit 11 bis 17 Kohlenstoffatomen und X Wasserstoff oder RC(O) ist, wobei mindestens eine der Gruppen X Wasserstoff ist und m einen Durchschnittswert von 1 bis 5 hat.

2. Gemisch von Oligomeren nach Anspruch 1, dadurch gekennzeichnet, daß m den Durchschnittswert 1 hat.

3. Gemisch von Oligomeren nach Anspruch 2, dadurch gekennzeichnet, daß durchschnittlich 3 bis 5 Gruppen X Wasserstoff sind.

4. Gemisch von Oligomeren nach einem der vorhergehenden Ansprüche, dadurch gekennzeichnet, daß R eine normale gesättigte oder ungesättigte Alkylgruppe mit 11 bis 15 Kohlenstoffatomen ist.

5. Gemisch von Oligomeren nach Anspruch 1 bis 4, dadurch gekennzeichnet, daß RCO eine Lauroyl-, Myristoyl-, Palmitoyl-, Stearoyl- oder Oleoylgruppe ist.

6. Verfahren zum Erzeugen eines Gemisches von Oligomeren nach Anspruch 1, in dem Pentaerythritol mit 0,5 bis 2,5 mol einer normalen gesättigten oder ungesättigten Fettsäure mit 12 bis 18 Kohlenstoffatomen verestert und das teilveresterte Produkt einer säurekatalysierten Kondensation zum Ausscheiden von 0,66 bis 0,85 mol Wasser pro mol Pentaerythritol unterworfen wird.

7. Verfahren nach Anspruch 6, dadurch gekennzeichnet, daß durch die Kondensation etwa 0,66 mol Wasser pro mol Pentaerythritol ausgeschieden wird.

8. Verfahren nach Anspruch 6 oder 7, dadurch gekennzeichnet, daß die Teilveresterung mit einer Säure, die aus der Laurin-, Myristin-, Palmitin-, Stearin- und Ölsäure ausgewählten Säure in einer Menge von 1 bis 2 mol pro mol Pentaerythritol bewirkt wird.

9. Zusammensetzung, die für ein topisches Auftragen auf die Haut geeignet ist und in einer anfeuchtend oder erweichend wirksamen Menge ein Oligomerengemisch aus teilverestertem Pentaerythritol nach einem der Ansprüche 1 bis 5 enthält.

10. Zusammensetzung nach Anspruch 9, die außerdem einen für kosmetische Zwecke geeigneten Emulgator enthält.

11. Zusammensetzung nach Anspruch 10, dadurch gekennzeichnet, daß der Emulgator aus der Gruppe ausgewählt ist, die aus den Aminsalzen von Fettsäuren, den Monoestern des Glycerins mit Fettsäure, den gegebenenfalls polyalkoxylierten Fettalkoholen mit 12 bis 20 Kohlenstoffatomen und den polyalkoxylierten Estern von Fettsäuren mit 12 bis 20 Kohlenstoffatomen besteht.

12. Zusammensetzung nach Anspruch 11, dadurch gekennzeichnet, daß das Oligomerengemisch aus teilverestertem Pentaerythritol ein Ester ist, in dem 0,5 bis 2,5 der Hydroxylgruppen des Pentaerythritols mit einer Fettsäure verestert werden sind, die aus der Laurin-, Myristin-, Stearin-, Palmitin- und Ölsäure ausgewählt sind.

13. Zusammensetzung nach Anspruch 12, die ferner ein erweichendes Mittel enthält.

14. Zusammensetzung nach Anspruch 10, die ferner ein erweichendes Mittel enthält, das aus der Gruppe ausgewählt ist, die aus den acetylierten Lanolinalkoholen, dem Isopropylmyristat, dem Isopropylpalmitat und Mineralöl besteht.

**Revendications**

1. Mélange d'oligomères de formule:

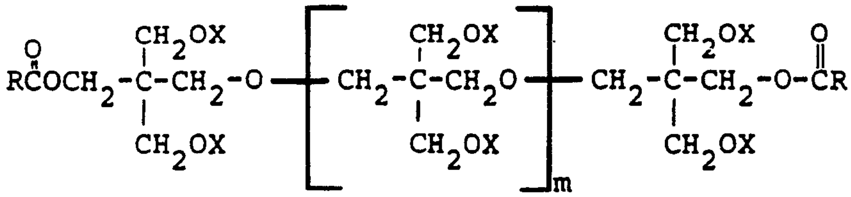

dans laquelle R est un radical alkyle normal saturé ou insaturé de 11 à 17 atomes de carbone, X est un atome d'hydrogène ou un groupe RC(O), à la condition qu'au moins l'un des groupes X soit un atome d'hydrogène et la valeur moyenne de *m* est de 1 à 5.

2. Mélange d'oligomères selon la revendication 1, dans lequel la valeur moyenne de *m* est de 1.

3. Mélange d'oligomères selon la revendication 2, dans lequel une moyenne de 3 à 5 groupes X sont des atomes d'hydrogène.

4. Mélange d'oligomères selon l'une quelconque des revendications précédentes, dans lequel R est un radical alkyle normal saturé ou insaturé de 11 à 15 atomes de carbone.

5. Mélange d'oligomères selon les revendications 1 à 4, dans lequel RCO est un radical lauryle, myristyle, palmityle, stéaryle ou oléyle.

6. Procédé de préparation d'un mélange d'oligomères selon la revendication 1, qui consiste à l'estérification de pentaérythritol avec 0,5 à 2,5 moles d'un acide gras normal saturé ou insaturé de 12 à 18 atomes de carbone et une condensation catalysée par un acide de l'ester partiel obtenu afin d'extraire de 0,66 à 0,85 mole d'eau par mole de pentaérythritol.

7. Procédé selon la revendication 6, dans lequel on effectue la condensation de façon à extraire environ 0,66 mole d'eau par mole de pentaérythritol.

8. Procédé selon la revendication 6 ou 7, dans lequel on effectue l'estérification partielle avec 1 à 2 moles par mole de pentaérythritol d'un acide choisi parmi les acides laurique, myristique, palmitique, stéarique et oléique et leurs mélanges.

9. Composition appropriée pour application locale à la peau, qui comprend une quantité efficace humidifiante ou émolliente d'un mélange d'oligomères de pentaérythritol partiellement estérifié tel que défini dans l'une quelconque des revendication 1 à 5.

10. Composition selon la revendication 9, qui contient en outre un émulsifiant cosmétiquement acceptable.

11. Composition selon la revendication 10, dans laquelle ledit émulsifiant est choisi parmi les sels d'amines des acides gras, les monoesters de glycérol avec un acide gras, les alcools gras de 12 à 20 atomes de carbone qui sont facultativement polyalcoxylés et les esters polyalcoxylés d'acides gras de 12 à 20 atomes de carbone.

12. Composition selon la revendication 11, dans laquelle ledit mélange d'oligomères de pentaérythritol partiellement estérifié est un ester dans lequel 0,5 à 2,5 groupes hydroxy du pentaérythritol ont été estérifiés avec un acide gras qui est choisi parmi les acides laurique, myristique, stéarique, palmitique ou oléique.

13. Composition selon la revendication 12, qui comprend en outre un émollient.

14. Composition selon la revendication 10, qui contient en outre un émollient choisi parmi les alcools de lanoline acétylée, le myristate d'isopropyle, le palmitate d'isopropyle et les huiles minérales.